(19) Europäisches Patentamt / European Patent Office / Office européen des brevets

(11) **EP 4 202 956 A1**

(12) # EUROPEAN PATENT APPLICATION

(43) Date of publication:
**28.06.2023 Bulletin 2023/26**

(21) Application number: **21217672.1**

(22) Date of filing: **24.12.2021**

(51) International Patent Classification (IPC):
*G16H 50/30* (2018.01)   *G16H 40/67* (2018.01)

(52) Cooperative Patent Classification (CPC):
**G16H 50/30; G16H 40/67**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **WIRTH, Christoph Tobias**
  **5656 AG Eindhoven (NL)**
• **SOKORELI, Ioanna**
  **5656 AG Eindhoven (NL)**
• **PAUWS, Steffen Clarence**
  **5656 AG Eindhoven (NL)**

(74) Representative: **Philips Intellectual Property & Standards**
**High Tech Campus 52**
**5656 AG Eindhoven (NL)**

(54) **COPD MONITORING**

(57)    A system and method is provided for allocating a patient to one of a plurality of COPD remote patient monitoring programs, each remote patient monitoring program being tailored to a different level of disease acuity.

**FIG. 4**

EP 4 202 956 A1

**Description**

FIELD OF THE INVENTION

The present invention relates to the field of monitoring chronic obstructive pulmonary disease (COPD), and in particular to the field of allocating patients to COPD remote patient monitoring (RPM) programs.

BACKGROUND OF THE INVENTION

Telehealth and remote patient monitoring solutions aim to reduce the frequency and severity of diseases exacerbations, to improve the quality of life of patients and to reduce resource use and cost for the health system. For example, telehealth and remote patient monitoring systems are used for managing patients with COPD.

COPD patients of different acuity levels have different care needs and require RPM programs tailored to these needs. Need-tailored RPM programs for COPD have the potential to reduce emergency department visits and admissions.

The total cost of care of treating COPD patients increases with disease severity. While medication cost is the main cost driver for the lower disease severity stages (e.g., GOLD stages 1 and 2) inpatient cost becomes the main cost driver for progressed disease severity stages (e.g., GOLD stages 3 and 4). A successful RPM program must aim at stabilizing patients for a longer time period in their current disease severity stage, so that a rapid increase in treatment cost can be averted. GOLD refers to the Global Initiative for Chronic Obstructive Lung Disease.

There is a clear need in optimizing RPM programs for COPD. One issue is that patients need to be placed in an RPM program optimized for the patients' acuity levels. For RPM programs to be effective, a set of program levels must be defined where each program level targets an acuity level effectively. There is a need for a robust way to determine the acuity levels of a patients and thereby allocate patients to the correct RPM program level.

SUMMARY OF THE INVENTION

The invention is defined by the claims.

According to examples in accordance with an aspect of the invention, there is provided a system for allocating a patient to one of a plurality of chronic obstructive pulmonary disease, COPD, remote patient monitoring, RPM, programs, each RPM program being tailored to a different level of disease acuity, the system comprising a processor adapted to:

receive an indication of disease severity of the patient, and thereby derive a disease severity level;
receive an assessment of the symptomatic state of the patient to estimate a symptom burden level of COPD symptoms;
estimate a future outcome level that is at least indicative of next-year cost of care;
receive geriatric assessment data on the patient to estimate a level of frailty;
combine the levels of disease severity, symptom burden, frailty and future outcome to derive a total patient score; and
determine an acuity level in order to allocate the patient to the corresponding RPM program based on at least the total patient score.

The system implements a data-driven method to guide patients into the most appropriate RPM program using a patient score. This score-based initial RPM program allocation (with the option of a subsequent dynamic update) allows that the overall eligible COPD cohort is effectively managed by a multi-stage RPM program for a defined set of acuity levels. RPM program stages offer varying degrees of vital signs monitoring, coaching and patient engagement including self-management and provision of educational content.

For example, each patient is assessed based on components relating to symptom burden e.g. the GOLD ABCD grade, the disease severity, e.g. the GOLD 1234 stage, the estimated next-year cost of care, frailty and optionally vital signs using a scoring mechanism to arrive at a total patient score (which may be considered to be a total COPD acuity score). The total patient score is then transformed to determine an acuity level of the patient that is then used to recommend the corresponding RPM program level) using a transformation function.

The estimated cost of care are those costs which would be incurred by the patient without allocation to an RPM program - in other words the level associated with such costs (e.g., low, moderate, high, very high) are determinant of the future state of the patient, since the total cost of care (not including the cost for RPM monitoring itself) is indicative of a general degree of health of the patient. Instead of predicting the next-year cost of care directly, a predicted future surrogate outcome such as hospitalizations or re-admission risks can be taken to indicate the patient's predicted cost level.

The eventual recommendation of a particular RPM program level may be for a specific time period based on the total patient score or a transformation of this score, but also based on additional information relating to the clinical profile of the patient and pre-set capacity targets for RPM program allocation. The score is designed to identify the right RPM program level at initial enrolment. The method can however also be used in dynamic re-assessment of whether the patient is still allocated to the right RPM program level or should be enrolled into a RPM program level which addresses the patient's score-determined new acuity level.

The system may be used to stratify a COPD population of an insurer or care provider into different RPM program levels according to the care needs of each acuity level. Each RPM program level can require different levels of patient management. For example, a low acuity program can contain patient engagement with self-management, a medium acuity program adds care management and a high acuity program has an additional case management component. Patients with severe COPD would benefit more from a high intensity RPM program while patients with mild or moderate COPD would benefit from a lower intensity RPM program.

The processor is for example adapted to receive vital signs measurements of available devices to estimate a level of vital signs status, and combine the total patient score with the vital signs level or with other score components to update the total patient score.

The total patient score is then built on five components (i) diagnosed COPD severity, (ii) symptom burden and exacerbation history, (iii) future outcome incorporating at least the expected cost of care without an RPM program, (iv) estimated frailty and (v) vital signs measurements of available devices. The method is based on self-reported data that can be acquired through surveys.

The patient score, its transformations, estimated acuity levels, as well as the recommended RPM program levels, may be displayed to the medical professional, either on a case-by-case patient level, or on a population level using a list ordered by acuity level. The display is for example in the form of an allocation chart which visualizes component scores for disease severity, symptom burden, predicted outcome (e.g. direct medical cost), frailty and vital signs and other patient-related information. An output metric may be provided such as a score, an odds, a probability or an acuity level (and a corresponding recommended RPM program).

The indication of disease severity of the patient for example comprises a GOLD 1234 stage, wherein the processor is adapted to allocate each stage a score.

Estimating the cost of care (as part of the future outcome) for example comprises using a generalized linear model to derive a predicted cost of care. The processor is then for example adapted to allocate a cost score to the predicted cost of care.

The symptom burden assessment for example comprises a GOLD ABCD grade assessment. This assessment takes into account the exacerbation history and the COPD assessment test (CAT) score, or an exacerbation history and the dyspnea grade measured by the mMRC (modified Medical Research Council dyspnea grade). The processor is adapted to allocate a symptom burden score to the symptom assessment.

The frailty component for example comprises a measure based on age and morbidity burden of the patient. The processor is then for example adapted to allocate a frailty score to frailty component.

Thus, scores are allocated to each of the score components; disease severity, symptom burden, cost of care, frailty and available vital signs. Additional factors may be provided, such as a score derived from other measurements. A total patient score then comprises the sum or a weighted sum of the different score components.

The processor is for example adapted to transform the total patient score into a transformed score using a transformation function, and apply thresholds to the transformed score to determine an acuity level.

The processor may be adapted to adapt the transformation function based on an assessment of whether historical initial allocations to a RPM program using the system were considered appropriate.

Deriving a total patient score may further comprise taking account of risk factors of COPD including one or more of:

    age;
    gender;
    education level;
    smoking status;
    body-mass-index;
    COPD assessment test (CAT);
    mMRC grade;
    presence of sputum;
    presence of cough;
    presence of dyspnea;
    health-related quality of life;
    years since diagnosis of COPD;
    health literacy;
    prescribed medications;
    previous healthcare utilizations (e.g., hospitalizations, ED visits);

presence of long-term oxygen therapy (LTOT);
presence of noninvasive ventilation (NIV);
comorbidities.

These risk factors in particular may form input features of the cost prediction model which then produces the cost score component of the total patient score.

The invention also provides a computer-implemented method for allocating a patient to one of a plurality of chronic obstructive pulmonary disease, COPD, remote patient monitoring programs, RPMs, each RPM program being tailored to a different level of disease acuity, the method comprising:

receiving an indication of disease severity of the patient, and thereby derive a disease severity level;
receiving an assessment of the symptomatic state of the patient to estimate the symptom burden level of COPD symptoms;
estimating a future outcome level that is at least indicative of next-year cost of care;
receiving geriatric assessment data on the patient to estimate a level of frailty;
combining the levels of disease severity, symptom burden, frailty and future outcome to derive a total patient score; and
determining an acuity level in order to allocate the patient to the corresponding RPM program based on at least the total patient score.

The method may comprise:

receiving vital signs measurements of available devices to estimate a level of vital signs status; and
combining the total patient score with the vital signs level or with other score components to update the total patient score.

The invention also provides a computer program comprising computer program code means which is adapted, when said computer program is run on a computer, to implement the method defined above.

The invention also provides a processor which is programmed to implement the computer program. The processor is for example a processor of a clinical decision support system.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 is a diagram depicting RPM programs for different acuity levels;
Figure 2 depicts a method which may be used by a system which also incorporates the invention;

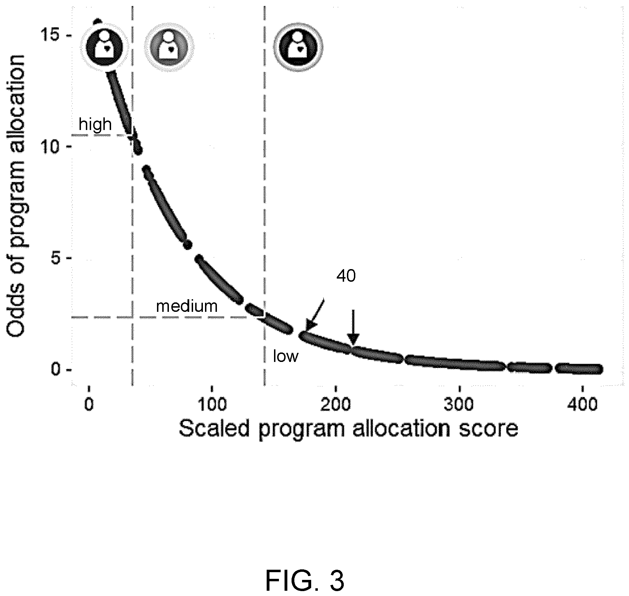

Figure 3 shows a relationship between a scaled program allocation score and program allocation odds;
Figure 4 depicts a method according to the invention;
Figure 5 shows the GOLD classification according to GOLD 1234 stages;
Figure 6 shows the GOLD classification according to GOLD ABCD grades;
Figure 7 shows a transformation from a program allocation score to program allocation odds;
Figure 8 shows a plot of the program allocation odds versus the scaled program allocation score with threshold values;
Figure 9 shows a display output; and
Figure 10 is a simplified block diagram of a computer within which one or more parts of an embodiment may be employed.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for allocating a patient to one of a plurality of remote patient monitoring programs, RPMs, each remote patient monitoring program being tailored to a different level of COPD disease acuity. Based on an indication of disease severity of the patient, a disease severity level is derived. Assessment of the symptomatic state of the patient is received to determine the burden of COPD symptoms and a future outcome level for the patient is predicted indicative of a next-year total cost of care. Based on patient information, a frailty level is determined. Available vital signs measurements are optionally also received to determine the vital signs level. The levels of disease severity level, symptom burden, outcome predictive of cost of care, frailty and (if used) other available vital signs levels are combined to derive a total patient score indicative of the patient's acuity level, and this is used to determine the COPD RPM program to which a patient should be allocated.

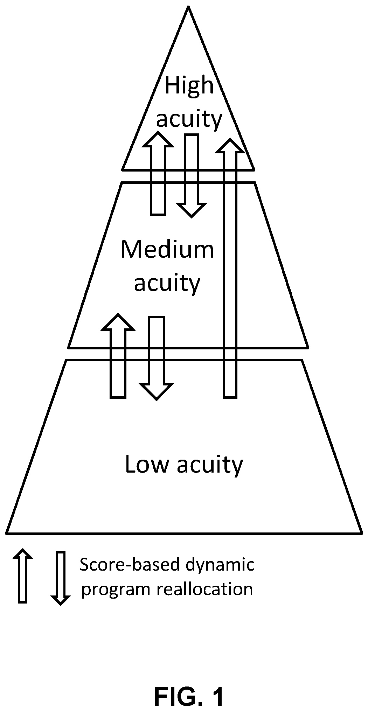

Figure 1 depicts different RPM programs related to different acuity levels of COPD disease.

In an example, for the low acuity program there may be personalized self-management with a COPD action plan and patient reported outcome measures (PROMs). There may be a patient education module to educate the patient about the symptoms of COPD and preventative measures. In a low acuity program, a patient may use their own device to manually enter data. On the basis of the PROMs, or other factors, the patient may initiate a request for primary care services.

A medium acuity program may include many, or all, of the support and monitoring features of a low acuity program but have additional support and monitoring. In an example of a medium acuity program there may again be a personalized self-management plan but instead of manual assessment data reporting and entry there may be automated data entry. In addition to patient-initiated requests for primary care services there may be scheduled healthcare appointments for example with a physician.

A high acuity program may include many, or all of the support and monitoring features but also have additional ones. There will be additional patient engagement and managed connected devices. There may be proactive intervention by telehealth services either in response to assessment data or at predetermined or random intervals.

Each patient is initially assessed for the disease acuity and placed into one of these three RPM programs based on the assessment of disease acuity. The patient remains on that RPM program for a certain time period or until the end of the RPM program regardless of the progress of the disease. It may be that a patient enrolled in a low acuity program deteriorates and would benefit from the additional monitoring and support available in a higher level program. This can affect the health and quality of life of a patient because a higher level RPM program may identify problems earlier allowing preventative measures to be taken, thus avoiding more severe incidences and interventions.

Conversely a patient may be in a high level RPM program and their health has improved. The benefit of the RPM program is therefore very low because the level of monitoring and support offered is not necessary.

A first concept which has been devised by the inventors is to allow patients to be enrolled into the optimum acuity level of RPM program for any given point in time. This approach can also help to relieve problems related to RPM program capacity: a RPM program may be full, thereby preventing the enrolment of new patients, even though the benefit to some patients currently enrolled in this RPM program is low. By recurrent periodic re-evaluation of the acuity level of patients, they can be removed from unsuitable or inappropriate RPM programs (and enrolled in another RPM program) thus releasing capacity for patients for whom the RPM program would be more beneficial.

A recurrent re-assessment of a patient condition and re-allocation to the right RPM program can thus be performed. As can be seen from Figure 1, a patient can move between acuity level RPM programs: more often between RPM programs of adjacent acuity but in more extreme cases directly from a low acuity program to a high acuity program. The transition between different RPM programs can occur at any time and is based on the acuity level and care needs of the patient.

Figure 1 is illustrative and there may be more than three levels of RPM program and the present invention is applicable to any number of levels of RPM program.

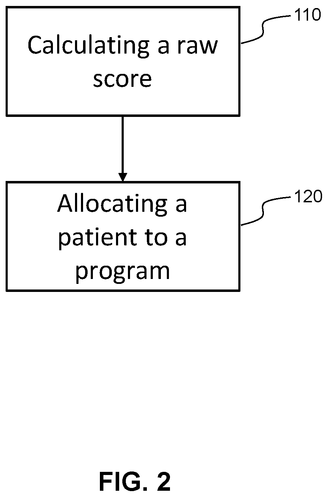

The dynamic allocation method which has been proposed by the inventors is depicted in Figure 2 and comprises calculating in step 110 a score based on one or more assessments and in step 120 allocating a patient to a COPD RPM program based on at least the total score. There may be a variety of assessments at different moments in time, and each assessment moment produces a score which can be used to allocate the patient to the appropriate RPM program level at that moment of time.

Each assessment moment involves collecting acuity measures from a patient. In an example, a set of measures is used which comprises:

   a symptom burden measure (e.g. incorporating a measure of a number of exacerbation events);
   a disease severity measure;
   a predictive model outcome indicative of cost-of-care;
   a measure for frailty; and

vital signs measurements (e.g. an oxygen saturation level).

On the basis of the resulting total score, a patient is allocated, or reallocated to a specific RPM program. The patient could be moved to a higher level program or a lower level program or remain in the same program if the patient was allocated to a program prior to the assessment moment. Indeed a patient may be moved to a RPM program at a non-adjacent level.

Different types of assessment may be used: some may measure the absolute value, whereas some may assess a change, or trend over time.

The COPD Assessment Test (CAT) is a self-reported patient questionnaire used to measure the overall impact of COPD on a patient's health status. The assessment takes into account symptoms including cough, sputum, dyspnea and chest tightness. A CAT assessment comprises a series of statements and the patient indicates how much the statement describes them. As an example, relating to the symptom of coughing, a patient would indicate where on a scale of 0-5 they fit with I never cough being 0 and I cough all the time being 5. In one example CAT there are eight different questions. The CAT score is the total of all the questions. For a CAT with eight questions there is a maximum score of 40, which would indicate that COPD has a very severe impact on the patient's quality of life.

An assessment of symptom burden for example includes a count of how many exacerbations there are in a predetermined period. An exacerbation is defined as an event leading to an admission to a hospital for COPD or two or more events which required a visit to a GP or medications but no hospitalization. There needs to be a predetermined period over which the occurrence of exacerbations is examined (e.g., the previous 12 months). The exacerbations may be reported by the patient or taken from electronic medical records (EMR) or claims data. A preferred example of a disease symptom burden measure is the GOLD (Global initiative for Chronic Obstructive Lung Disease) ABCD grade.

A preferred example of a disease severity measure is the GOLD 1234 stage . This is a method of classifying how severe COPD is for a patient and this can be used as an assessment for generating a raw score. There are four classifications (1234) which are based on the most recent spirometric assessment or alternatively the FEV1/FEV6 ratio measured by a handheld device. FEV1 indicates the amount of air forcefully exhaled in the first second of a six second test. FEV6 indicates the amount of air exhaled over the whole six seconds. The ratio FEV1/FEV6 gives an indication of the respiratory health of the patient with a higher result indicating better health.

More generally, disease severity can be derived from diagnostic spirometric assessment outcome metrics (e.g. GOLD 1234 stage, FEV1%, FEV1% predicted). It can be self-reported (e.g. questionnaire, mMRC grade), assessed via diagnostic classification coding system (e.g. ICD codes), or by physiological monitoring (e.g. handheld device measuring FEV1/FEV6), in particular by:

1. GOLD 1234 stage
2. FEV1% defined as FEV1/FVC ratio, also called Tiffeneau-Pinelli index
3. FEV1% predicted, which is defined as FEV1% of the patient divided by the average FEV1% for a person with similar characteristics
4. FEV1/FEV6 as a surrogate for FEV1/FVC measured by a handheld device
5. Diagnostic ICD codes for COPD (e.g. for ICD-10 classification of COPD codes J44.x.y with 3rd digit x (x = 0, 1, 8, 9) and 4th digit y (y = 0, 1, 2, 3, 9)
6. Severity grade of the Modified British Medical Research Council (mMRC) grade 0-IV

As an example of a vital signs measurement, oxygen saturation can also be used as an assessment for a raw score. The most recent assessment of arterial blood oxygen saturation of the patient is used. This may be measured using a pulse oximetry device. The assessment may use the following three categories for measured oxygen levels:

Very low: $SpO_2 \leq 90\%$
Low: $91\% \leq SpO_2 \leq 94\%$
Normal: $SpO_2 \geq 95\%$

Various different assessments are described above although the invention is not limited thereto and there may be other assessments additionally included.

This invention relates in particular to the allocation of patients to the correct RPM program at initial enrollment, and provides a scoring algorithm to calculate a total patient score (which may be considered to be an acuity score) and support the initial allocation of patients to the right RPM program.

The total patient score is initially designed to identify the right RPM program at enrolment. The method can however also be used for dynamic re-assessment of whether the patient is still allocated to the right RPM program or should be enrolled into a RPM program which addresses the patient's new level of acuity. However, the method for initial allocation considers slowly changing information as input features, whereas a subsequent dynamic allocation method may be based on self-reported symptom changes and can be used to confirm a new symptomatic state of the patient resulting in an RPM program level change.

The total patient score is transformed into a probability and odds and threshold values are provided which are used to determine a recommended RPM program for a newly enrolled patient. The invention thus relates to the initial triaging of patients to the different RPM programs. Data is collected prior to the start of the RPM program and a score is produced to allocate the patient to the correct RPM program.

The patient is typically meant to stay in this RPM program for a substantial time (e.g. 3 months before a re-assessment is done) as mentioned above. This is because higher acuity RPM programs make use of devices which cannot be handed out or recollected on a weekly or even daily basis. For that reason the method makes use of data which has a relatively slow rate of change.

For example, for this initial triaging process, different data categories may be used than for a subsequent dynamic assessment.

As a minimum, the invention makes use of a diagnostic disease severity (e.g. GOLD 1234 stage), a symptom burden (e.g. GOLD ABCD grade), and a predicted next-year cost of care level. The method may also be used to fill RPM programs by pre-defined capacity limits.

In a preferred implementation, the patient score is composed of five components:

(i) diagnosed COPD severity;
(ii) symptom burden;
(iii) expected cost of care without an RPM program;
(iv) estimated frailty and
(v) vital signs.

The method can be based on self-reported data that can be acquired through surveys. The vital signs component can be excluded from the model score if patients do not have a measuring device (e.g., pulse oximeter).

The predicted next-year total cost of care is considered as an input feature of the score because high cost patients are assumed to benefit from a higher acuity program due to higher complexity in care needs which drives health care cost of these patients. The predicted next-year total cost of care is an indication of what acuity level a patient would have if the patient was not allocated to any RPM program. Predicted next-year total cost of care is hence a measure for a future patient's state of health. Cost of care refers to claims reimbursed by health insurances for provision of health care services and prescribed medications.

A first component is the disease severity and it relates to a baseline acuity level measured by diagnostic severity. Thus, the processor of the system receives an indication of a disease severity of the patient, and thereby derives a baseline acuity level. This is the previously diagnosed disease severity.

To score the disease severity, the GOLD stage classification of airflow limitation severity may be used (based on post-bronchodilator FEV1) in patients with FEV1/FVC < 0.7. Other disease severity measures can be used. Thus, the disease severity may be obtained by spirometry or an alternative measure, and for example has values of 1, 2, 3 or 4.

A second component relates to the present symptom burden. Symptom burden is a composite measure, for example taking into account the presence of exacerbations in the past 12 months together with a self-reported outcome, for example of a CAT or mMRC questionnaire.

Thus, the processor periodically receives COPD symptom burden data relating to the COPD symptom burden. This is an indication of current symptom burden and (recent) exacerbation history. It may also take any value, and in the particular example shown below the values may be 1, 2, 3 or 4.

The third component scores the predicted future outcome which can be expressed by (total) cost of care or a cost component for treating COPD, such as medication cost, outpatient or inpatient cost or it can be related to future clinical utilization, such as hospitalizations, readmissions. This component can make use of existing multi-dimensional prognostic tools (e.g. to predict readmission risk). In this way, the processor predicts a future disease outcome for the patient and estimates a cost of care associated with the predicted future disease outcome. The predicted outcome is a next-year total cost of care. Next-year total cost of care can for example be derived by a generalized linear model with Gamma family and log link (multiplicative arithmetic mean model). The predicted cost of care level is for example presented as a value of 1, 2, 3 or 4.

The cost component is an aggregated cost for outpatient, inpatient and medication. The predictor variables for the GLM model are the GOLD severity stage, age, education level, smoking status, body-mass index, comorbidity count, presence of dyspnea, sputum, cough and exacerbation severity amongst others. The expected next-year cost level is determined by the GOLD 1234 stage.

If, however, the predicted next-year cost exceeds the upper limit of the 95%-confidence interval (CI) for the patient's expected cost, the patient's assigned cost level moves up by one level. Patients in GOLD stage 4 may always be assigned to the highest cost level - this is as the disease is progressive and there is not a cure.

The predicted next-year total cost of care is considered as an input feature of the score because high cost patients are assumed to benefit from a higher acuity RPM program due to higher complexity in care needs which drives health care cost of these patients.

The estimated cost of care may not be a direct cost estimate. It may for example instead be any predicted outcome which influence the cost of care. For example, a prediction may be made of clinical utilization (such as readmission risk, hospital admission risk). The predicted outcome will still have an associated cost can thus be mapped to predicted cost score.

For example, a predicted outcome may be a readmission risk. If a patient has a predicted readmission risk of 0.7, this might be classified as very high and hence the highest predicted cost score is set for this patient, e.g. level 4.

A fourth component is a multimorbidity or frailty index. This may be based on the age and morbidity count of the patient. The value may be normalized to an interval [0,1] as a continuous range. The age and morbidity burden is used as a proxy for estimated frailty.

One example makes use of a frailty score obtained by taking the expected mean age of the enrolled population, which might be 70 years. The actual age of a patient may then be divided by the mena (e.g. 70) and normalized to a value in the range [0,1].

The comorbidities of the patient are then counted and divided by an expected number e.g. 3. This score is then normalized.

The age-normalized score is then added to the comorbidity-normalized score so that the sum is in the range of [0,2], which is then further normalized to a range of [0,1].

Frailty is then the difference to a standard patient of 70 years with 3 morbidities: An older patient has a higher score (due to limitations of older age) and a patient with more morbidities has a higher score due to more complexity of care needs.

Alternative measures of frailty can be used, e.g. electronic frailty index (eFI) or Clinical Frailty Scale (CFS)

The (optional) fifth component is a SpO2 saturation level obtained using a pulse oximeter. The level is for example presented as a value 0,2 or 4. Note that this vital signs monitoring is however optional. If patients do not have a measuring device e.g., pulse oximeter then the vital signs component is not included in the score computation, so it is optional. In this example, the maximum total score of all sub scores is 13 without the vital signs and 17 with vital signs. The components may all be self-reported, acquired through surveys.

The individual components are each allocated a (raw) score, as described in more detail below. Thus, there is disease severity score corresponding to the, a cost score corresponding to the cost of care and a symptom burden score and a frailty score. There is optionally a vital signs score depending on availability of measuring devices.

A total patient score is obtained as the sum or a weighted sum of the individual scores.

Table 1 below shows an example of possible score components. (*95% upper confidence interval (CI) limits for expected annual total cost of care per GOLD stage 1-4; cost values adjusted to year 2019.)

| Score component | Description | Computation rule |
|---|---|---|
| **Disease severity** (GOLD stages 1234 by %FEV1 predicted) | Stage 1 = FEV1 $\geq$ 80% (mild COPD) | Gold score = 1 |
| | Stage 2 = FEV1 < 80% (moderate COPD) | Gold score = 2 |
| | Stage 3 = FEV1 < 50% (severe COPD) | Gold score = 3 |
| | Stage 4 = FEV1 < 30% (very severe COPD) | Gold score = 4 |
| Symptom burden (GOLD grades ABCD by exacerbation history and symptoms) | Grade A = exacerbations $\leq$ 1, less symptoms | Clinical score = 1 |
| | Grade B = exacerbations $\leq$ 1, more symptoms Grade C = exacerbations $\geq$ 2 or admission, less symptoms Grade D = exacerbations $\geq$ 2 or admission, more symptoms | Clinical score = 2 Clinical score = 3 Clinical score = 4 |
| **Predicted outcome** (next-year total cost of care) | Expected next-year cost category per GOLD stage; upgrade by 1 category if pred. cost exceeds upper 95% confidence interval limit Category 1 (+1 if pred. cost > € 5697*) for GOLD stage 1 Category 2 (+1 if pred. cost > € 6144*) for GOLD stage 2 Category 3 (+1 if pred. cost > € 9015*) for GOLD stage 3 Category 4 (set to highest cost category) for GOLD stage 4 | Predicted next-year cost score (if pred. cost > expected cost) Cost score = 1 (then 2) Cost score = 2 (then 3) Cost score = 3 (then 4) Cost score = 4 |

(continued)

| Score component | Description | Computation rule |
| --- | --- | --- |
| **Estimated frailty** | Assumes mean age amongst frail of e.g. 70 years; | Estimated frailty score = normalized ((age / average age) + (morbidities /average condition count)) |
| | and presence of 3 chronic conditions in average. age and morbidity sub-score each normalized to range (0,1). Frailty score normalized to (0,1) | |
| **Vital signs** | Use SpO2 saturation as optional input if patients have device | Optional |
| | SpO2 $\leq$ 90% (very low $O_2$ saturation) | SpO2 score = 4 |
| | 91% $\leq$ SpO2 $\leq$ 94% (low $O_2$ saturation) | SpO2 score = 2 |
| | SpO2 $\geq$ 95% (normal $O_2$ saturation) | SpO2 score = 0 |
| **Total Score** | Sum of all 4 score components (Score range 3 to 17 on continuous scale, Score range 3 to 13 on continuos scale without Vital signs) | Gold score + clinical score + cost score + frailty score + SpO2 score |

The score computation algorithm will now be explained. The score computation follows the following steps:

Step 1: Define model parameters.

The user selects the following values:

$\alpha_0$ is user-specified to define the baseline value for the probability *Pr* from (Eq. 2), e.g., 0.05
*shape* user-specified to define the shape of probability curve *Pr* from (Eq. 2), e.g. 2.5
*pdo* is the user-specified points-to-double the odds, e.g. 50
$points_0$ is determined so that max(odds) = odds (Score $\square$ 0) (optimization of Eq. 3)
cost model beta-coefficients $\beta_p$ e.g. according to Table 2 below.

Program allocation thresholds are selected according to a method described below.
The program allocation threshold levels are derived via a two-step calibration process which consists of

(i) an initial collection phase of score data elements and patient-level score computation from a reference population and
(ii) a score stratification so that the user-defined program capacity limits are matched.

Capacity limits on acceptable patient volume per RPM program are typically set by the user (e.g. health insurer or the RPM operations center) offering these RPM programs to eligible patients. Capacity limits also ensure that the RPM programs do not exceed the available budget for deploying the RPM program.

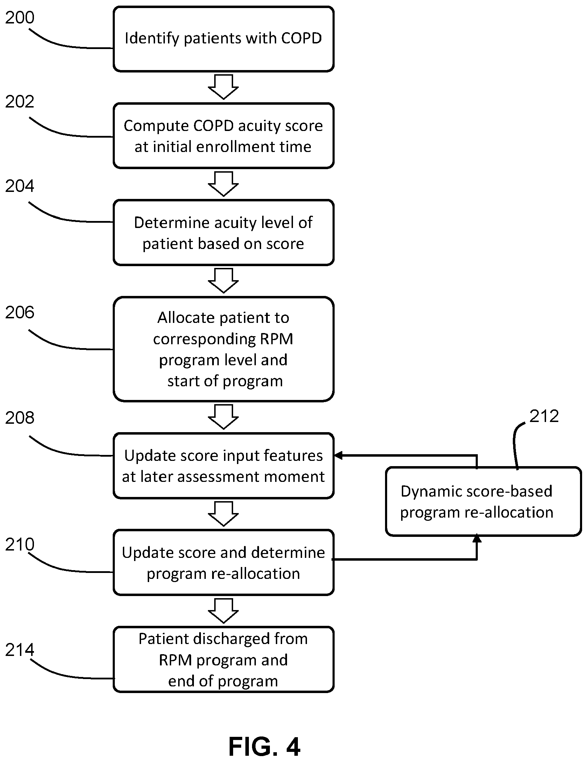

Figure 4 shows program allocation odds verses the scaled program allocation score. It relates to the program allocation odds and scaled allocation score for a total cohort of 2,000 patients. (Parameters: $\alpha_{Pr}$ = -2.94, $\beta_{Pr}$ = 0.57, offset = 204, pdo = 50)
In Figure 4, each dot 40 represents a patient. The user-selected program capacity limits (75% of patients allocated to low acuity program, 20% of patients allocated to medium acuity program and 5% of patients allocated to high acuity program) then define the score strata for program allocation.
In this case, the low-medium threshold is set to an odds of 2.37 and the medium-high threshold to an odds of 10.50 which corresponds to 142.3 and 34.9 points on the scaled score. If for example for a newly enrolled patient the odds are computed to 11 (which is larger than 10.50) this patient is recommended for the high acuity program.

Step 2: Collect and store data from each patient.

Patient-specific information can be collected via questionnaires deployed via a patient-facing app or website or via other clinical data sources or any other sources.

Step 3. For each patient *i*, compute the expected next-year cost.

This may be performed by a multiplicative arithmetic mean generalized linear model (GLM) with Gamma family and log link according to:

$$(\text{Eq. 1}) \qquad E[Cost_i] = Cost_0 \cdot \prod_p \exp(\beta_p \cdot x_{p,i}) \leftrightarrow \ln E[Cost_i] = \beta_0 + \boldsymbol{\beta} \cdot \boldsymbol{X_i}$$

The predicted cost of care is for example for the next year and may include outpatient costs, inpatient costs and medication.

The model parameters $\beta_p$ are initialized by training of a above model on available cost data and predictor data (predictors see Table 2 below).

An initial binary parameter set is also described in Byng D, et al. "Determinants of healthcare utilization and costs in COPD patients: first longitudinal results from the German COPD cohort COSYCOT", 2019, Int J Chron Obstruct Pulmon Dis 14, 1423-1439.

Table 1. Cost prediction model coefficients. *CI = Confidence Interval

| Predictor | $\beta_p$ (95% CI*) |
|---|---|
| (Intercept) $\beta_0$ | 2301 (1758 - 3012) |
| GOLD II | 0.22 (0.07 - 0.36) |
| GOLD III | 0.24 (0.09 - 0.39) |
| GOLD IV | 0.45 (0.24 - 0.66) |
| Age 65-74 | 0.22 (0.07 - 0.35) |
| Age > 74 | 0.18 (0.01 - 0.36) |
| Higher education | -0.11 (-0.22 - 0.00) |
| Smoker | 0.17 (0.00 - 0.35) |
| Underweight (BMI<18.5) | 0.50 (0.25 - 0.76) |
| Count of comorbities > 3 | 0.40 (0.32 - 0.48) |
| Presence of dyspnea (mMRC grade $\geq$ 2) | 0.26 (0.17 - 0.34) |
| Presence of moderate exacerbation in past 12 months | 0.20 (0.10 - 0.29) |
| Presence of severe exacerbation in past 12 months | 0.55 (0.44 - 0.66) |

Step 4: For each patient, compute total score

This is performed using Table 1 above

Step 5: Compute the estimated acuity probability Pr and odds based on the logistic function.

The probability and odds values are computed by the following transformations:

$$(\text{Eq.2}) \qquad Pr = \frac{exp(\alpha_{Pr} + \beta_{Pr} \cdot Score)}{1 + exp(\alpha_{Pr} + \beta_{Pr} \cdot Score)} \leftrightarrow odds = \frac{Pr}{1 - Pr} = exp(\alpha_{Pr} + \beta_{Pr} \cdot Score)$$

$$Score = Total\ Score - \underbrace{\min(Total\ Score)}_{=3} \quad \alpha_{Pr} = \ln\left(\frac{\alpha_0}{1 - \alpha_0}\right) \quad \beta_{Pr} = \frac{1}{shape \cdot \ln 2}$$

where:

Step 6: Compute a scaled score based on scale.

This is performed using a transformation of the logit value of the probability *Pr* by:

(Eq. 3)
$$\text{scaled score} = \text{offset} - \frac{\text{pdo}}{\ln 2} \cdot \underbrace{\text{logit}(Pr)}_{=ln(odds)} = \text{offset} - \frac{\text{pdo}}{\ln 2} \cdot (\alpha_{Pr} + \beta_{Pr} \cdot \text{Score})$$

$$offset = \text{points}_0 - \frac{\text{pdo}}{\ln 2} \cdot \ln(odds_0) \quad odds_0 = \frac{1}{\exp(\alpha_{Pr})}$$

where

By the above scale transformation, the program allocation odds now double at every reduction on the scaled score by the parameter *pdo* (points-to-double the odds). In other words, the higher the odds the higher the determined acuity level of the patient. The acuity level now changes by the same factor for every constant amount in scaled score points. The program allocation is now derived from the *odds* or equivalenty from the *scaled score.*

The overall algorithm is schematically shown in Figure 4. It may be used in conjunction with a system providing dynamic allocation of RPM programs as explained above.

In step 200, patients with COPD are identified.

Patient cohort data is obtained and imported for storage and further use. Patient data is for example sourced from repositories as a hospital EMR, ambulatory EMR, GP practice system or an insurance claims administrative data system. Data sources can be linked by various entities: patient identifier, event identifier, location identifier, coding elements including diseases/diagnoses (ICD-9/10 codes), medical procedures (HCPCS/CPT codes) and prescriptions to provide this complete picture.

In general, a data input module queries the selected data sources to extract all the medical status information of patients based on demographics and medical history information relating to diagnoses, procedures, symptoms, patient-reported outcomes and vital signs. The medical status information is queried and stored on a case-by-case basis. In the case that claims management system data is received, medical status information is read from the EHR directly.

In step 202 a COPD acuity score is computed. The acuity score reflects the current acuity level of patients based on all selected score components available at enrollment. This makes use of an AI module.

In step 204 the patient score is converted into a nominal acuity level (e.g. low, medium, high).

The determined acuity levels then identify and recommend patients as candidates to participate in the respective RPM programs for designed for high, medium and low acuity levels.

Step 206 consists of the actual allocation and onboarding of a patient to the RPM program level. This step is performed by a clinical decision support (CDS) system which includes an implementation of the herein described algorithm in a software application that executes on a processor of a computer. This step can further involve the clinical assessment of the RPM program recommendation by a clinical provider, assessment of eligibility to the RPM program, optionally contact with the patient for a shared decision about participating to the RPM program and if patient agrees enrollment to the RPM program and providing any support needed including for example RPM program instructions, app, devices. This algorithm can be implemented in software accessing patient records in GP (ambulatory, out-patient) software systems, hospital EMR systems or administrative claim data from health insurer or care provider systems or on patient-reported data.

The patient is then enrolled in the RPM program.

A dynamic reallocation optionally takes place in steps 208 and 210 as explained above. In step 208, the assessments explained above are received and a patient score is updated in step 210. A dynamic score-based RPM program reallocation then takes place in step 212 periodically, until the patient is discharged from the RPM program or at the terminal end of the RPM program is reached in step 214.

The score update in step 210 may be based on any suitable combination of measures deterministic of a patient's acuity level.

The initial allocation in step 206 is that which is described in greater detail above, and provides the automated and optimized initial RPM program recommendation for the COPD patients. It is based on cost and clinical parameters providing a holistic assessment of current acuity level of the patient at the time of enrollment. It provides improved efficiency in allocating patients to the right RPM program. The system can be implemented with low cost and is applicable in any clinical setting such as RPM operations centers, primary care, hospital, or health insurances. To identify eligible COPD patients data from GP ambulatory systems, in-patient EMR or administrative claims data from insurance can be used. It requires very limited self-reported information, and provides an increase in specificity (and hence precision) of follow-up care provided to the patient if enrolled to the right RPM program.

The initial allocation thus involves identifying patients in the population, collecting a minimum set of required self-reported data, and using an AI module to generate a RPM program allocation recommendation. The recommendation can be provided by a clinical decision support (CDS) system adapted with a computer implementation of the herein described RPM program allocation algorithm. The recommendation can in turn be evaluated by the clinical provider so that a shared decision with the patient is reached about the right RPM program, following which the patient is enrolled, and optionally a dynamic reallocation system may be used as explained above.

The method is flexible so can be adjusted or calibrated to the cohort at hand. The cases with a confirmed high acuity in the cohort express the mean prior probability or prevalence of high-acuity COPD patients in the cohort allowing to correct for over- or under-estimation of acuity levels in a population. The method can also be adjusted to correct for over- or under-exaggeration of COPD acuity in a population, that is cases with a high confirmed acuity that would receive a too low (or too high) acuity estimate and cases with a low confirmed acuity that would receive a too high (or too low) acuity estimate.

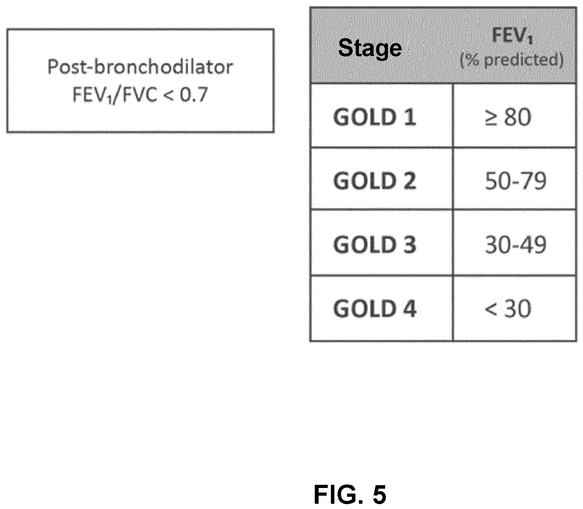

Figure 5 shows one example of how the GOLD disease severity stages are derived. After a spriometrically confirmed diagnosis, an assessment of airflow limitation is obtained for patients with a value of FEV1/FVC < 0.7. The severity stage is then obtained from the FEV1% predicted level.

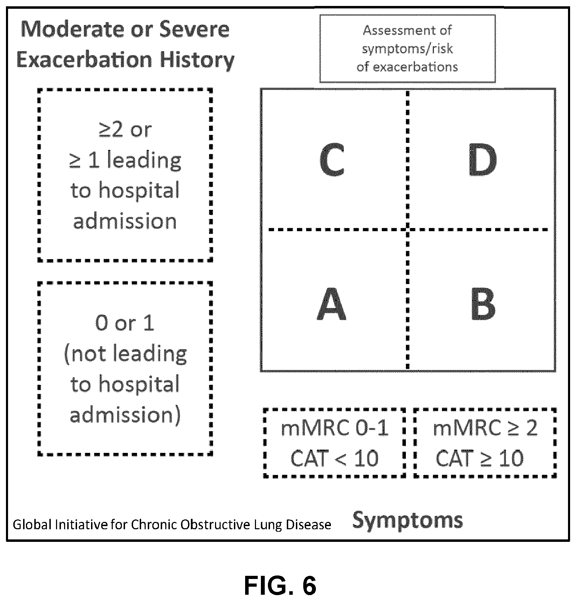

Figure 6 shows one example of how the GOLD grades for assessing symptom burden are derived, based on the combination of the exacerbation history (number of exacerbation events and how many lead to hospital admission) and the COPD Assessment Test (CAT) score or by taking the exacerbation history and the grading of dyspnea (the modified Medical Research Council, mMRC, dyspnea scale, which is a self-rating measure of the degree of disability caused by breathlessness). If exacerbation history and mMRC assessment are not available in any of the available patient records, the patient may be approached to self-report this information.

The CAT score is a self-reported patient questionnaire used to measure the overall impact of COPD on a patient's health status. The assessment takes into account symptoms including cough, sputum, dyspnea and chest tightness. A CAT assessment comprises a series of statements and the patient indicates how much the statement describes them. As an example, relating to the symptom of coughing, a patient would indicate where on a scale of 0-5 they fit with I never cough being 0 and I cough all the time being 5. In one example CAT there are eight different questions. The CAT score is the total of all the questions. For a CAT with eight questions there is a maximum score of 40, which would indicate that COPD has a very severe impact on the patient's quality of life.

The mMRC scale has a range 0 to 4 for different levels of breathlessness and the consequent impact on the patient.

As explained above, a log odds value is used to create a scaled program allocation score such that every fixed number of point increase (e.g., 50 points) of the scaled program allocation score means an actual doubling of the odds for program allocation. In particular, the odds then doubles at every reduction on the scaled score by a parameter pdo (points to double the odds). Program triaging can then take place using the scaled score or using the odds before scaling.

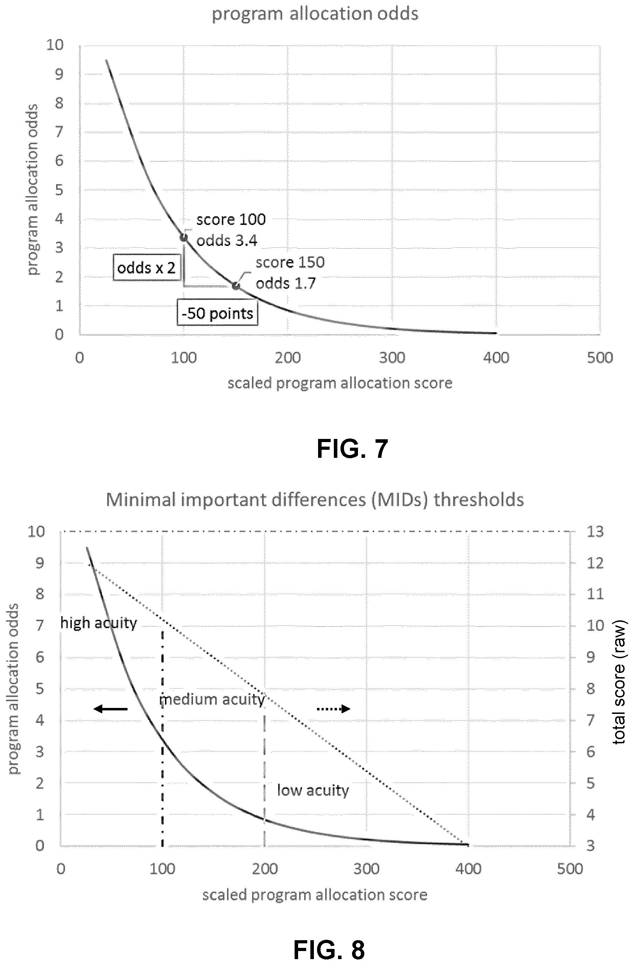

This transformation is shown in Figure 7. It shows the program allocation odds versus the scaled program allocation score. In this way, a re-scaled version of the raw score is transformed in such a way that every 50 point decrease means a doubling in odds.

The truncation thresholds may be applied on the scaled score or equivalently they can be applied on the odds. Each patient has an odds and a scaled score so a threshold on the scaled score determines the threshold for odds.

Patients are then assigned to an acuity level which is then used for the initial COPD RPM program allocation. For assigning patients to an acuity category, thresholds are applied to indicate high, medium or low acuity for COPD.

Truncation thresholds (i.e. boundaries between different determined acuity levels using the scaled program allocation score or equivalently on the odds) are applied to the scaled program allocation score as multiples of the doubling of the odds.

Figure 8 shows a plot of the program allocation odds versus the scaled program allocation score with the thresholds, which can be adjusted. In Figure 8, the scaled program allocation score has a start value of 400 and truncation thresholds at score points of 350, 300, 250, 200, 150, 100, 50.

The dotted sloped line in Figure 8 refers to the total raw score which is just the sum of the five sub-scores and hence extends across the range from 3 to 13 (assuming no vital signs measurement).

Each threshold defines a patient group with a group variation of odds of a factor of 2 when the points the odds double is set to 50. In this example, the odds range is from 0-10, and the odds can be converted to an acuity level. The odds and corresponding acuity levels can then be grouped up into acuity categories (high, medium, low).

Truncation threshold levels are for example defined in a way that a minimal important difference (MID) must separate adjunct threshold levels. This means that the difference between truncation threshold must be clinically meaningful to justify an allocation to a RPM program of a different level.

The thresholds are for example set so that the correct numbers of patients are initially applied to each RPM program, to fit the desired ratio of patients to the different RPM programs.

In Figure 8, the MID for the high/medium threshold is set to 100 points on the scaled program allocation score and for the medium/low MID threshold 200 points. These MID thresholds can then be used for allocation to COPD RPM programs.

However, the chosen threshold values are arbitrary but dictate the specificity, sensitivity and precision of the program-screening algorithm. It is possible to maximize specificity at the expense of sensitivity (or vice versa) that is better in "ruling out" ("ruling in") higher severity for COPD.

In another embodiment, other thresholds can be taken that might result from an in-depth analysis (receiver operating characteristics analysis, decision curve analysis).

In an example, the targets (or limits) for RPM program allocation are pre-defined. These express what proportion of patients with COPD need to be allocated to each RPM program. A typical definition is 5% (high acuity), 20% (medium acuity) and 75% (low acuity). Thus, the majority of patients (75%) need to be allocated to low-acuity programs, and only a small amount (5%) need to be allocated to high-acuity programs. These ratios can be achieved by setting the truncation levels as explained above.

Threshold values can be estimated that respect these targets, when allocating patients to RPM programs.

Various methods exist to present the acuity information to the end user, i.e. the medical professional. Each patient case or record can be annotated with a total patient score, acuity estimate and its acuity level (high, moderate, low). The patient score can be explained by the presence of risk factors as identified from the data. By opening or printing the patient record, this annotation will help the medical professional to actively identify the relevant characteristics of the patient driving the risk score.

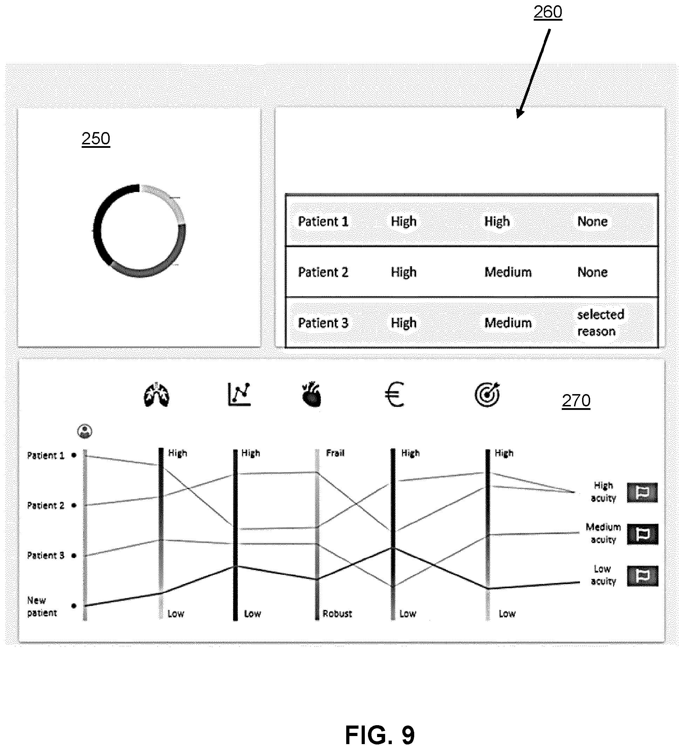

For example, telehealth coaches of an RPM operations center use an allocation dashboard as part of a computer-implemented clinical decision support (CDS) system which can be used to visualize an AI-enabled initial recommendation of a patient into one of the three program acuity levels. An example dashboard is shown in Figure 9.

The current distribution of patients to RPM programs is shown in region 250. In region 260, for each patient the current RPM program, the recommended RPM program and a reason for a program change is shown.

The graphs in region 270 show the combination of raw scores for each patient and the resulting RPM program recommendation. Program allocation odds may be shown.

Multiple patient cases can thus be ordered and displayed list-wise on total acuity estimate from high to low acuity. The cases at the top of the list are the cases with highest acuity who are recommended to be enrolled to more intensive programs. The information presented in the CDS-based allocation dashboard can for example be used by the medical professional together with the patient to arrive at the final joint decision of the RPM program that the patient should be enrolled to.

When implementing the method, some cases can disqualified from further analysis such as patients without insurance coverage, patients with a terminal illness or in need of palliative or hospice care, patients who suffer from pre-defined, non-COPD-related diseases (malignant neoplasms, intellectual disabilities, Parkinson or Alzheimer), patients who undergo specific costly therapies for severe conditions (chemo/radiation therapy for cancer treatment, dialysis, long-term ventilation) and patients who await a lung transplant.

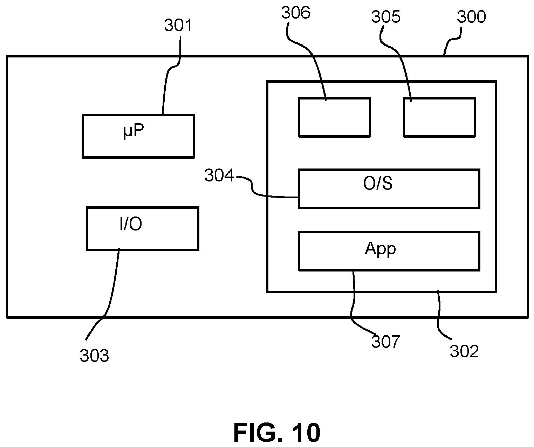

Figure 10 illustrates an example of a computer 300 within which one or more parts of an embodiment may be employed. Various operations discussed above may utilize the capabilities of the computer 300. For example, one or more parts of a system for providing a subject-specific user interface may be incorporated in any element, module, application, and/or component discussed herein. In this regard, it is to be understood that system functional blocks can run on a single computer or may be distributed over several computers and locations (e.g. connected via internet).

The computer 300 includes, but is not limited to, PCs, workstations, laptops, PDAs, palm devices, servers, storages, and the like. Generally, in terms of hardware architecture, the computer 300 may include one or more processors 301, memory 302, and one or more I/O devices 303 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 301 is a hardware device for executing software that can be stored in the memory 302. The processor 301 can be virtually any custom made or commercially available processor, a central processing unit (CPU), a digital signal processor (DSP), or an auxiliary processor among several processors associated with the computer 300, and the processor 301 may be a semiconductor based microprocessor (in the form of a microchip) or a microprocessor.

The memory 302 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 302 may incorporate electronic, magnetic, optical, and/or other types of storage media. Note that the memory 302 can have a distributed architecture, where various components are situated remote from one another, but can be accessed by the processor 301.

The software in the memory 302 may include one or more separate programs, each of which comprises an ordered listing of executable instructions for implementing logical functions. The software in the memory 302 includes a suitable operating system (O/S) 304, compiler 306, source code 305, and one or more applications 307 in accordance with exemplary embodiments. As illustrated, the application 307 comprises numerous functional components for implementing the features and operations of the exemplary embodiments. The application 307 of the computer 300 may represent various applications, computational units, logic, functional units, processes, operations, virtual entities, and/or modules in accordance with exemplary embodiments, but the application 307 is not meant to be a limitation.

The operating system 304 controls the execution of other computer programs, and provides scheduling, input-output control, file and data management, memory management, and communication control and related services. It is contemplated by the inventors that the application 307 for implementing exemplary embodiments may be applicable on all commercially available operating systems.

Application 307 may be a source program, executable program (object code), script, or any other entity comprising a set of instructions to be performed. When a source program, then the program is usually translated via a compiler (such as the compiler 306), assembler, interpreter, or the like, which may or may not be included within the memory 302, so as to operate properly in connection with the O/S 304. Furthermore, the application 307 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 303 may include input devices such as, for example but not limited to, a mouse, keyboard, scanner, microphone, camera, etc. Furthermore, the I/O devices 303 may also include output devices, for example but not limited to a printer, display, etc. Finally, the I/O devices 303 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 303 also include components for communicating over various networks, such as the Internet or intranet.

If the computer 300 is a PC, workstation, intelligent device or the like, the software in the memory 302 may further include a basic input output system (BIOS) (omitted for simplicity). The BIOS is a set of essential software routines that initialize and test hardware at startup, start the O/S 304, and support the transfer of data among the hardware devices. The BIOS is stored in some type of read-only-memory, such as ROM, PROM, EPROM, EEPROM or the like, so that the BIOS can be executed when the computer 300 is activated.

When the computer 300 is in operation, the processor 301 is configured to execute software stored within the memory 302, to communicate data to and from the memory 302, and to generally control operations of the computer 300 pursuant to the software. The application 307 and the O/S 304 are read, in whole or in part, by the processor 301, perhaps buffered within the processor 301, and then executed.

When the application 307 is implemented in software it should be noted that the application 307 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 307 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed processing method may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

## Claims

1. A system for allocating a patient to one of a plurality of chronic obstructive pulmonary disease, COPD, remote patient monitoring programs, RPMs, each RPM program being tailored to a different level of disease acuity, the system comprising a processor (300) adapted to:

   receive an indication of disease severity of the patient, and thereby derive a disease severity level;
   receive an assessment of the symptomatic state of the patient to estimate the symptom burden level of COPD symptoms;
   estimate a future outcome level that is at least indicative of next-year cost of care;
   receive geriatric assessment data on the patient to estimate a level of frailty;
   combine the levels of disease severity, symptom burden, frailty and future outcome to derive a total patient score; and
   determine an acuity level in order to allocate the patient to the corresponding RPM program based on at least the total patient score.

2. The system of claim 1, wherein the processor is adapted to receive vital signs measurements of available devices to estimate a level of vital signs status, and combine the total patient score with the vital signs level or with other score components to update the total patient score.

3. The system of claim 1 or 2, wherein the indication of disease severity of the patient comprises a GOLD 1234 severity stage, wherein the processor is adapted to allocate each stage a disease severity score.

4. The system of any one of claims 1 to 3, wherein estimating a future outcome level comprises using a generalized linear model to derive a predicted next-year cost of care.

5. The system of claim 4, wherein the processor is adapted to allocate a cost score to the predicted next-year cost of care.

6. The system of any one of claims 1 to 5, wherein the symptom burden level comprises a GOLD ABCD grade.

7. The system of claim 6, wherein the processor is adapted to allocate a symptom burden score to the symptom burden level.

8. The system of claims 3, 5 and 7, wherein the total patient score comprises the sum or a weighted sum of the disease severity score, the cost score, the symptom burden score, and a frailty score.

9. The system of any one of claims 1 to 8, wherein the processor is adapted to transform the total patient score into a transformed score using a transformation function, and apply thresholds to the transformed score to determine the acuity level.

10. The system of claim 9, wherein the processor is adapted to adapt the transformation function based on an assessment of whether historical initial allocations to a RPM program using the system were considered appropriate.

11. The system of any one of claims 1 to 10, wherein deriving a total patient score further comprises taking account of risk factors of COPD including one or more of:

    age;
    gender;
    education level;
    smoking status;
    body-mass-index;

COPD assessment test (CAT);
mMRC grade;
presence of sputum;
presence of cough;
presence of dyspnea;
health-related quality of life;
years since diagnosis of COPD;
health literacy;
prescribed medications;
previous healthcare utilizations (e.g., hospitalizations, ED visits);
presence of long-term oxygen therapy (LTOT);
presence of noninvasive ventilation (NIV);
comorbidities.

12. A computer-implemented method for allocating a patient to one of a plurality of chronic obstructive pulmonary disease, COPD, remote patient monitoring programs, RPMs, each RPM program being tailored to a different level of disease acuity, the method comprising:

receiving an indication of disease severity of the patient, and thereby derive a disease severity level;
receiving an assessment of the symptomatic state of the patient to estimate the symptom burden level of COPD symptoms;
estimating a future outcome level that is at least indicative of next-year cost of care;
receiving geriatric assessment data on the patient to estimate a level of frailty;
combining the levels of disease severity, symptom burden, frailty and future outcome to derive a total patient score; and
determining an acuity level in order to allocate the patient to the corresponding RPM program based on at least the total patient score.

13. The method of claim 12, comprising:

receiving vital signs measurements of available devices to estimate a level of vital signs status; and
combining the total patient score with the vital signs level or with other score components to update the total patient score.

14. A computer program comprising computer program code means which is adapted, when said computer program is run on a computer, to implement the method of claim 12 or 13.

15. A processor which is programmed to implement the computer program of claim 14, wherein the processor is a processor of a clinical decision support system.

High
acuity

Medium
acuity

Low acuity

Score-based dynamic
program reallocation

## FIG. 1

Calculating a raw score — 110

Allocating a patient to a program — 120

**FIG. 2**

FIG. 3

**FIG. 4**

Post-bronchodilator
$FEV_1/FVC < 0.7$

| Stage | FEV$_1$ (% predicted) |
|-------|-----------------------|
| GOLD 1 | ≥ 80 |
| GOLD 2 | 50-79 |
| GOLD 3 | 30-49 |
| GOLD 4 | < 30 |

FIG. 5

Moderate or Severe Exacerbation History

Assessment of symptoms/risk of exacerbations

≥2 or
≥ 1 leading
to hospital
admission

0 or 1
(not leading
to hospital
admission)

C

D

A

B

mMRC 0-1
CAT < 10

mMRC ≥ 2
CAT ≥ 10

Global Initiative for Chronic Obstructive Lung Disease    Symptoms

FIG. 6

program allocation odds

FIG. 7

Minimal important differences (MIDs) thresholds

FIG. 8

260

250

| Patient 1 | High | High | None |
|-----------|------|------|------|
| Patient 2 | High | Medium | None |
| Patient 3 | High | Medium | selected reason |

270

| | | | | |
|---|---|---|---|---|
| Patient 1 | High | High | Frail | High | High |
| Patient 2 | | | | | |
| Patient 3 | | | | | |
| New patient | Low | Low | Robust | Low | Low |

High acuity

Medium acuity

Low acuity

FIG. 9

24

**FIG. 10**

Europäisches
Patentamt

European
Patent Office

Office européen
des brevets

# EUROPEAN SEARCH REPORT

Application Number

EP 21 21 7672

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/081866 A1 (PROTERIXBIO INC [US]) 23 April 2020 (2020-04-23) * abstract * * paragraph [0026] - paragraph [0040] * * paragraph [0073] - paragraph [0107] * * paragraph [0127] - paragraph [0196] * * paragraph [0243] - paragraph [0377] * * figure 33 * | 1-15 | INV. G16H50/30 G16H40/67 |
| A | HANNA SANDELOWSKY ET AL: "COPD - do the right thing", BMC FAMILY PRACTICE, BIOMED CENTRAL LTD, LONDON, UK, vol. 22, no. 1, 11 December 2021 (2021-12-11), pages 1-17, XP021300011, DOI: 10.1186/S12875-021-01583-W * the whole document * | 1-15 | |
| A | ANDREA VIANELLO ET AL: "Home telemonitoring for patients with acute exacerbation of chronic obstructive pulmonary disease: a randomized controlled trial", BMC PULMONARY MEDICINE, BIOMED CENTRAL LTD, LONDON, UK, vol. 16, no. 1, 22 November 2016 (2016-11-22), pages 1-12, XP021266764, DOI: 10.1186/S12890-016-0321-2 * the whole document * | 1-15 | TECHNICAL FIELDS SEARCHED (IPC) G16H |

The present search report has been drawn up for all claims

| Place of search | Date of completion of the search | Examiner |
|---|---|---|
| Munich | 31 May 2022 | Hilbig, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 21 7672

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2022

| Patent document cited in search report | Publication date | Patent family member(s) | Publication date |
|---|---|---|---|
| WO 2020081866 A1 | 23-04-2020 | US 2022003785 A1 | 06-01-2022 |
| | | WO 2020081866 A1 | 23-04-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

• **BYNG D et al.** Determinants of healthcare utilization and costs in COPD patients: first longitudinal results from the German COPD cohort COSYCOT. *Int J Chron Obstruct Pulmon Dis,* 2019, vol. 14, 1423-1439